# EUROPEAN PATENT SPECIFICATION

(11) **EP 0 876 170 B1**
(45) Date of publication and mention of the grant of the patent: **01.10.2003**
(21) Application number: 97904730.5
(22) Date of filing: 07.01.1997
(51) Int. Cl.: A61M 1/16

(54) **BLOOD OXYGENATOR WITH HEAT EXCHANGER**
BLUTOXYGENATOR MIT WÄRMEAUSTAUSCHER
OXYGENATEUR DE SANG AVEC ECHANGEUR THERMIQUE

(30) Priority: 11.01.1996 US 585322; 11.01.1996 US 586163; 11.01.1996 US 585324
(43) Date of publication of application: 11.11.1998
(73) Proprietor: MEDTRONIC, INC., Minneapolis, Minnesota 55432 (US)
(72) Inventor: ELGAS, Roger, J., Anaheim, CA 92802 (US); COREY, Edmund R. Jr., Spring Mountain Summit, Schwensville, PA 19473 (US); GREMEL, Robert, F., Huntington Beach, CA 92649 (US); HAMLEN, Robert, C., Edina, MN 55435 (US)
(74) Representative: Hughes, Andrea Michelle
(86) International application number: US9700069
(87) International publication number: WO97033636

(56) References cited:
- EP-A- 0 408 000
- EP-A- 0 507 722
- EP-A- 0 582 959
- WO-A-91/04758
- US-A- 4 196 075
- US-A- 4 622 206

## Description

This invention relates to surgical support apparatus, and more particularly, to an improved blood oxygenator used to maintain a patient's blood at a predetermined temperature while replacing carbon dioxide in the blood with oxygen.

Blood oxygenators are well known in the medical field. Typically they are disposable components of so-called "heart-lung machines". These machines mechanically pump a patient's blood and oxygenate the blood during major surgery such as a heart bypass operation. A typical commercially available blood oxygenator includes a heat exchanger and a membrane-type oxygenator. The patient's blood is continuously pumped through the heat exchanger. A suitable heat transfer fluid such as water is also pumped through the heat exchanger, separated from the blood but in heat transfer relationship therewith. The water is either heated or cooled externally of the blood oxygenator to maintain the patient's blood at a predetermined desired temperature. The membrane oxygenator comprises a so-called "bundle" of thousands of tiny hollow fibers made of a special polymer material having microscopic pores. Blood exiting the heat exchanger flows around the outside surfaces of these fibers. At the same time an oxygen-rich gas mixture, sometimes including anesthetic agents, flows through the hollow fibers. Due to the relatively high concentration of carbon dioxide in the blood arriving from the patient, carbon dioxide from the blood diffuses through the microscopic pores in the fibers and into the gas mixture. Due to the relatively low concentration of oxygen in the blood arriving from the patient, oxygen from the gas mixture diffuses through the microscopic pores in the fibers into the blood. The oxygen content of the blood is raised, and its carbon dioxide content is reduced. The blood is also heated or cooled before being returned to the patient.

EP-A-0408000 discloses a fluid processing apparatus having fluid inlets and fluid outlets and partitions for partitioning the housing interior into separate fluid chambers in repsective communication with the inlets and outlets.

EP-A-0582959 discloses a heat exchanger having corrugated surfaces cerating adjacent chambers for blood and water for efficient heat transfer.

A blood oxygenator must have a sufficient volumetric flow rate to allow proper temperature control and oxygenation. However, blood is typically in short supply and is very expensive. Therefore, it is desirable to minimize the volume of blood contained within the oxygenator, preferably to less than five hundred cubic centimeters. The cells and platelets in human blood are delicate and can be traumatized if subjected to excessive shear forces. Therefore, the blood flow velocity inside a blood oxygenator must not be excessive. In addition, the configuration and geometry of the inlet nozzle, manifolds and outlet nozzle of the blood flow path for a given blood flow rate must not create re-circulations (eddies) or stagnant areas that can lead to clotting.

It is common for a blood oxygenator to be positioned close to the floor of the operating room. Conventional blood oxygenators have either in-line or side-by-side heat exchangers and membrane oxygenators. This leads to undesirable height. Furthermore, if the blood is to enter the oxygenator vertically from beneath, it would be desirable for its blood inlet nozzle to be positioned so as to prevent kinking of the blood supply tube connected thereto. It is also important that the blood passing through the inlet nozzle be uniformly distributed throughout all of the conduits of the heat exchanger to maximize the heat exchange efficiency. Therefore, an inlet manifold with an optimum geometry and configuration is required.

Once the blood has flowed through the heat exchanger it needs to be redirected around the thousands of fibers of the membrane oxygenator in an efficient and uniform manner. This requires some sort of transition manifold with an optimum geometry and configuration.

After the blood has flowed around the fibers of the membrane oxygenator, it must be collected and routed outside the blood oxygenator in a uniform and efficient manner. This requires an optimally configured outlet manifold that couples to an outlet nozzle sized for connection to the standard flexible tubing that conveys the blood back to the patient.

It is therefore the primary object of this invention to provide an improved blood oxygenator.

It is another object of this invention to minimize the physical size of a blood oxygenator.

It is another object of this invention to minimize the internal volume of a blood oxygenator that must be filled with blood.

It is another object of this invention to provide a blood oxygenator with an improved blood flow path designed to minimize trauma to blood cells and platelets.

It is another object of this invention to provide a blood oxygenator with an improved blood flow path designed to minimize re-circulations and stagnant areas that could lead to clotting.

It is another object of this invention to provide a blood inlet manifold for a blood oxygenator which will minimize the physical size of the blood oxygenator.

It is another object of this invention to provide a blood inlet manifold for a blood oxygenator which will minimize the internal volume of the blood oxygenator that must be filled with blood.

It is another object of this invention to provide a blood inlet manifold for a blood oxygenator which minimizes shear forces that result in trauma to blood cells and platelets.

It is another object of this invention to provide a blood inlet manifold for a blood oxygenator designed to minimize re-circulations and stagnant areas that could lead to clotting.

It is another object of this invention to provide a transition manifold that is optimally configured for directing blood flowing out of a heat exchanger into a surrounding membrane-type oxygenator.

It is another object of this invention to provide an improved blood outlet manifold for a blood oxygenator.

It is another object of this invention to provide a blood outlet manifold for a blood oxygenator that will minimize the internal volume of the blood oxygenator that must be filled with blood.

It is another object of this invention to provide a blood outlet manifold for a blood oxygenator with a blood flow path designed to minimize trauma to blood cells and platelets.

It is another object of this invention to provide a blood outlet manifold for a blood oxygenator with a blood flow path designed to minimize re-circulations and stagnant areas that could lead to clotting.

According to one aspect of the present invention, there is provided a blood oxygenator, comprising: a heat exchanger including a housing and a plurality of conduits having first and second ends, and extending vertically through an interior of the housing; a first seal surrounding the exterior surfaces of the first ends of the conduits and a second seal surrounding the exterior surfaces of the second ends of the conduits, the first and second seals engaging an inner surface of the heat exchanger housing to prevent a heat transfer fluid flowing around the exterior surfaces of the conduits within the interior of the housing from mixing with blood flowing through the conduits; a heat transfer fluid inlet communicating with the interior of the heat exchanger housing; a heat transfer fluid outlet communicating with the interior of the heat exchanger housing; a blood inlet manifold for delivering blood from a first tube to the first ends of the conduits of the heat exchanger; a generally cylindrical oxygenator fiber bundle concentrically surrounding the heat exchanger; a vessel surrounding the oxygenator fiber bundle; a third seal surrounding the exterior surfaces of the first ends of a plurality of hollow fibers of the oxygenator bundle and a fourth seal surrounding the exterior surfaces of the second ends of the fibers of the oxygenator bundle, the third and fourth seals engaging the housing of the heat exchanger and the vessel to prevent blood flowing around the exterior surfaces of the fibers of the oxygenator bundle from communicating with the hollow interiors of the fibers of the oxygenator fiber bundle; a transition manifold for delivering blood from the second ends of the conduits of the heat exchanger around the exterior surfaces of the fibers of the oxygenator fiber bundle in a first portion of the oxygenator bundle; an outlet manifold for delivering blood from around the exterior surfaces of the fibers of the oxygenator fiber bundle in a second portion of the oxygenator fiber bundle to a second tube; an inlet header coupled to the vessel for directing a venous gas mixture from a first hose to the first ends of the hollow fibers of the oxygenator fiber bundle so that the gas mixture flows therethrough; and an outlet header coupled to the vessel for directing the venous gas mixture flowing out of the second ends of the hollow fibers of the oxygenator bundle to a second hose.

According to another aspect of the present invention, there is provided a method of oxygenating blood while maintaining a predetermined temperature thereof comprising the steps of: pumping blood in a first direction into the first ends of a plurality of vertical hollow heat exchanger conduits; pumping a heat transfer fluid around the outside surfaces of the heat exchanger conduits in a second direction opposite to the first direction to transfer heat between the fluid and the blood; directing the blood out of the second ends of the plurality of hollow conduits in a third direction substantially orthogonal to the first direction generally around a first end portion of the oxygenator fiber bundle and then in the second direction around the outside surfaces of a plurality of hollow fibers of a membrane-type oxygenator fiber bundle concentrically surrounding the heat exchanger conduits; introducing an oxygen-rich gas mixture into the first ends of the hollow fibers of the oxygenator fiber bundle; collecting oxygenated blood from around a second end portion of the oxygenator fiber bundle opposite the first end portion; and collecting a carbon-dioxide enriched gas mixture from the second ends of the hollow fibers of the oxygenator fiber bundle.

The blood inlet manifold distributes incoming blood from the inlet substantially uniformly over the plurality of micro-conduits for vertical flow upwardly parallel to a central vertical axis.

The transition manifold distributes the blood substantially uniformly around the plurality of fibers in an upper portion of the membrane oxygenator, after which the blood flows downwardly to a lower portion of the membrane oxygenator. The transition manifold minimizes shear forces that traumatize blood cells and platelets, and also minimizes recirculations which can lead to clotting.

Preferred embodiments of the invention will now be described by way of example only, with reference to the accompanying drawings. Throughout the drawing figures, like reference numerals refer to like parts.
Fig. 1 is an exploded isometric view of a blood oxygenator constructed in accordance with this invention.
Fig. 2 is a side elevation view of the blood oxygenator.
Fig. 3 is a top plan view of the blood oxygenator.
Fig. 4 is a diagrammatic view illustrating the blood, heat transfer fluid and gas mixture flow paths of the blood oxygenator.
Fig. 5 is a diagrammatic view illustrating the fabrication of the oxygenator fiber bundle of the blood oxygenator.
Fig. 6 is a diagrammatic view of the heat exchanger fiber bundle of the blood oxygenator.
Fig. 7 is an enlarged side elevation view of the spindle of the heat exchanger of the blood oxygenator around which is wound the micro-conduit wrapping material.
Fig. 8 is a cross-section view of the spindle of Fig. 7 taken along line 8-8 of Fig. 7.
Fig. 9 is an end elevation view of the spindle of Fig. 7 taken from the right end of Fig. 7.
Fig. 10 is an enlarged front elevation view of the blood inlet manifold of the blood oxygenator.
Fig. 11 is an enlarged rear elevation view of the blood inlet manifold of the blood oxygenator.
Fig. 12 is a vertical sectional view of the blood inlet manifold of the blood oxygenator taken along line 12-12 of Fig. 11.
Fig. 13 is a top plan view of the blood inlet manifold of the blood oxygenator.
Fig. 14 is a further enlarged, fragmentary vertical sectional view of illustrating portions of the conical wall member, vertical lip and rim of the blood inlet manifold of the blood oxygenator of Figs. 10-13.
Fig. 15 is an enlarged, fragmentary, broken away view illustrating the internal assembly of the components of the blood oxygenator.
Fig. 16 is an enlarged view of a portion of Fig. 15 illustrating details of the blood outlet manifold of the blood oxygenator.
Fig. 17 is a top plan view of the lower venous gas header of the blood oxygenator. Also visible in this figure are the inner heat exchanger housing, the water inlet nozzle, the water outlet nozzle and the gas mixture outlet nozzle.
Fig. 18 is a sectional view of the lower venous gas header and inner heat exchanger housing taken along line 18-18 of Fig. 17.
Fig. 19 is a sectional view of the lower venous gas header and inner heat exchanger housing taken along line 19-19 of Fig. 17.
Fig. 20 is a front elevation view of the lower venous gas header and inner heat exchanger housing of the blood oxygenator.
Fig. 21 is a side elevation view of the lower venous gas header and inner heat exchanger housing of the blood oxygenator.
Fig. 22 is an enlarged vertical sectional view of the lower venous gas header and the inner heat exchanger housing with the blood inlet manifold connected thereto. Also illustrated in this view is the micro-conduit fiber bundle of the heat exchanger.
Fig. 23 is an enlarged side elevation view of the outer heat exchanger housing and the transition manifold.
Fig. 24 is a vertical sectional view of the outer heat exchanger housing and the transition manifold taken along line 24-24 of Fig. 23.
Fig. 25 is a horizontal sectional view of the outer heat exchanger housing and transition manifold taken along line 25-25 of Fig. 23.

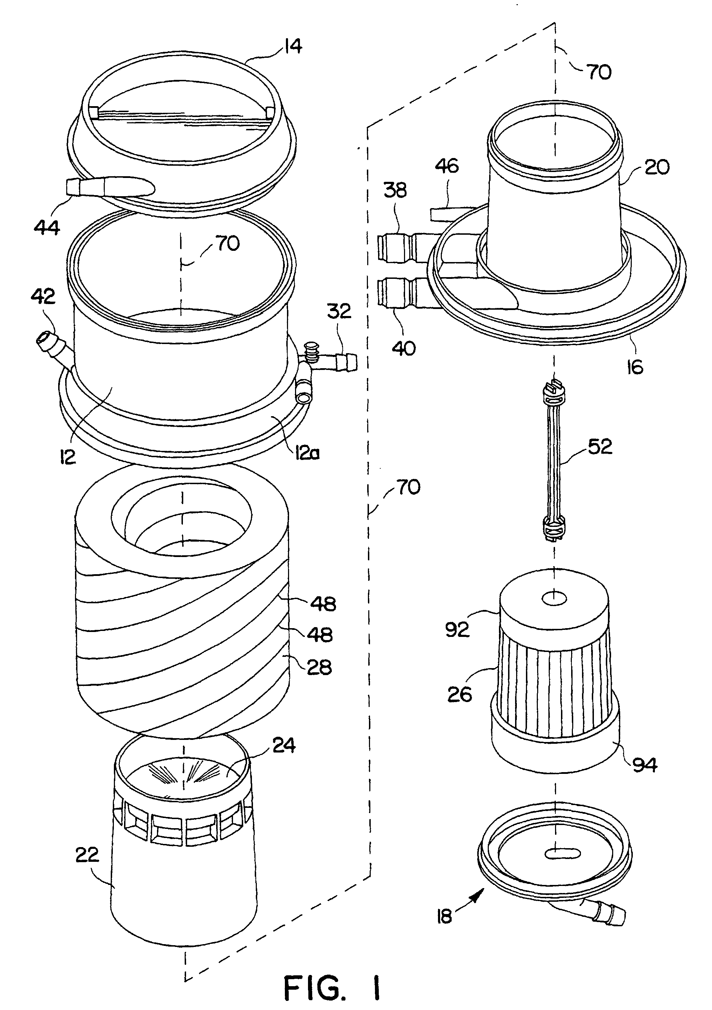

Referring to Figs. 1-3, a blood oxygenator 10 constructed in accordance with this invention comprises an outer generally cylindrical vessel 12 which is sealed at its upper end by a generally saucer-shaped upper hollow venous gas header 14. The lower end of the vessel 12 is sealed by a generally saucer-shaped lower hollow venous gas header 16. A blood inlet manifold 18 is connected to the center of the underside of the lower venous gas header 16. Concentric, generally cylindrical inner and outer heat exchanger housings 20 and 22 are connected at their lower ends to the center of the lower venous header 16. The upper end of the outer heat exchanger housing 22 includes a transition manifold 24. The interior of the inner heat exchanger housing 20 surrounds and encloses a generally cylindrical first fiber bundle 26 made up of a plurality of vertically oriented hollow micro-conduits. These micro-conduits convey blood vertically therethrough in an upward direction. A second generally cylindrical fiber bundle 28 concentrically surrounds the outer heat exchanger housing 22 and is positioned inside the inner wall of the cylindrical vessel 12. The upper and lower ends of the generally ring-shaped second fiber bundle 28 communicate with the upper and lower venous gas headers 14 and 16, respectively.

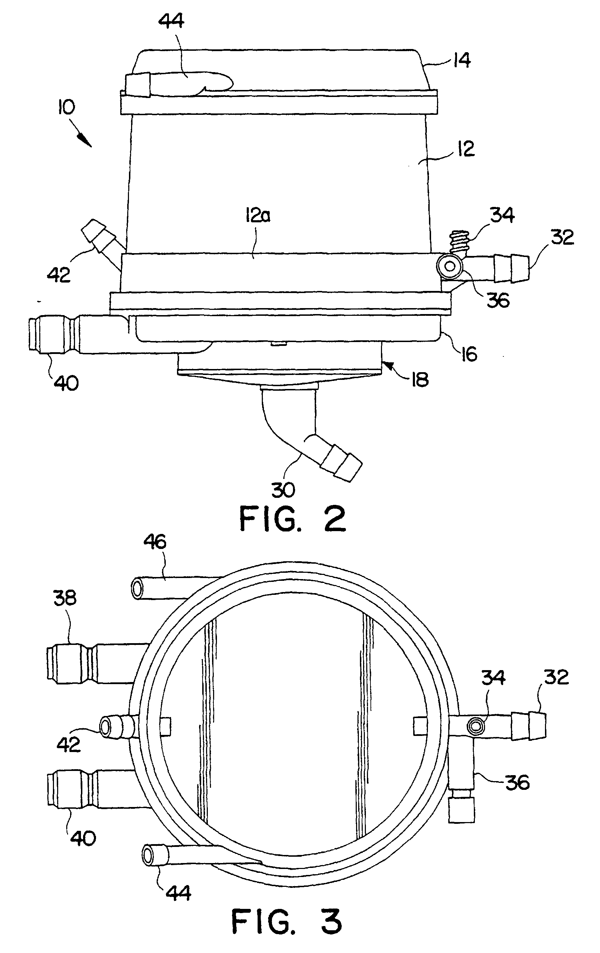

The blood inlet manifold 18 (Fig. 2) includes a barbed blood inlet nozzle 30 which bends downwardly at an angle relative to the central vertical axis of the vessel 12. A barbed blood outlet nozzle 32 (Figs. 2 and 3) extends horizontally from the exterior of an enlarged or flared portion 12a of the vessel 12. A standard luer fitting 34 (Fig. 2) extends vertically from the base of the blood outlet nozzle 32. A thermometer probe fitting 36 (Fig. 3) extends horizontally from the base of the blood outlet nozzle 32.

Inlet and outlet nozzles 38 and 40 (Figs. 1 and 3) for a heat transfer fluid such as water extend horizontally from one side of the low venous gas header 16 and communicate with water flow passages inside the inner heat exchanger housing 20. A barbed de-bubbler nozzle 42 (Fig. 2) extends upwardly at an angle from the flared portion 12a of the vessel 12. A gas mixture inlet nozzle 44 (Figs. 1, 2 and 3) extends horizontally from the periphery of the upper venous gas header 14. A gas mixture outlet nozzle 46 (Figs. 1 and 3) extends from the periphery of the lower venous gas header 16 parallel to the water inlet and outlet nozzles 38 and 40.

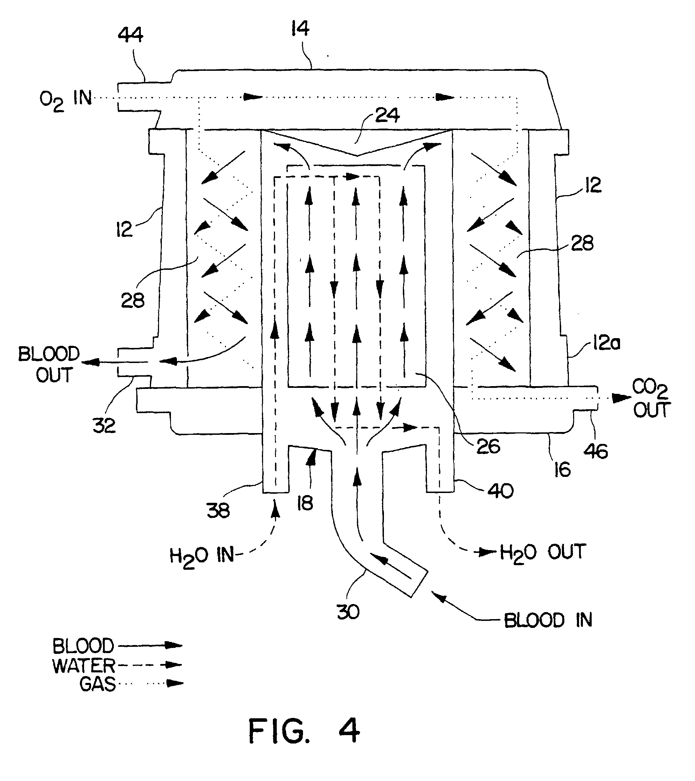

The blood, heat transfer fluid and gas mixture flow paths of the blood oxygenator 10 can best be understood by way of reference to the diagrammatic vertical sectional view of Fig. 4. In that figure, the flow of blood is illustrated diagrammatically by the bold solid arrows. The flow of heat transfer fluid (water) is illustrated by the dashed lines. The flow of gas mixture is illustrated by the sequence of dots. Blood from the patient flows through tubing (not illustrated) connected to the blood inlet nozzle 30. This incoming blood spreads out through the blood inlet manifold 18 and travels vertically in an upward direction through the micro-conduits of the first fiber bundle 26 of the central heat exchanger that forms the core of the blood oxygenator 10. Water flows in through the inlet nozzle 38 vertically upward to the top of the heat exchanger fiber bundle 26 through a separate channel isolated from the fiber bundle 26. The water is then directed downwardly and across the outside of the micro-conduits of the fiber bundle 26. The water flows around the outside of the micro-conduits in a direction opposite to the direction of flow of the blood within the micro-conduits. The water exiting from the lower end of the first fiber bundle 26 exits through the outlet nozzle 40. The water is heated or cooled outside the blood oxygenator, as necessary to regulate the temperature of the blood flowing through the micro-conduits of the heat exchanger. The use of a counter-flow heat exchanger provides optimum heat exchange efficiency. The temperature of the blood can be monitored by a circuit (not illustrated) that includes a thermistor or other temperature sensing device (not illustrated) mounted inside the thermometer probe fitting 36 (Figs. 2 and 3).

Blood exiting from the upper end of the first fiber bundle 26 (Fig. 4) of the heat exchanger is directed radially outwardly by the transition manifold 24. This blood then travels around the outside of the fibers of the second fiber bundle 28 that forms the membrane oxygenator. The blood travels downwardly past the outside surfaces of the fibers of the second fiber bundle 28. When the blood reaches the lower portion of the second fiber bundle 28, it is collected in an outlet manifold defined by the flared portion 12a of the vessel and exits through the blood outlet nozzle 32. The blood outlet nozzle 32 is connected to tubing (not illustrated) for returning the blood to the patient.

A gas mixture rich in oxygen from a pressurized source (not illustrated) is conveyed through a hose (not illustrated), through the gas mixture inlet nozzle 44, and into the upper venous gas header 14. The upper gas header 14 communicates with the upper ends of the fibers in the second fiber bundle 28 forming the membrane oxygenator. The oxygen-rich gas mixture travels down through the interior of the fibers in the fiber bundle 28. These fibers are micro-porous. Carbon dioxide from the blood surrounding the fibers in the bundle 28 diffuses through the walls of the fibers into the gas mixture. Similarly, oxygen from the gas mixture inside the fibers of the bundle 28 diffuses through the micropores into the blood. The gas mixture now having an elevated carbon dioxide content exits the lower ends of the fibers of the second fiber bundle 28 into the lower venous gas header 16 and then exits therefrom via the gas mixture outlet nozzle 46. This gas mixture now has a lowered oxygen content. The nozzle 46 is connected to another gas hose (not illustrated).

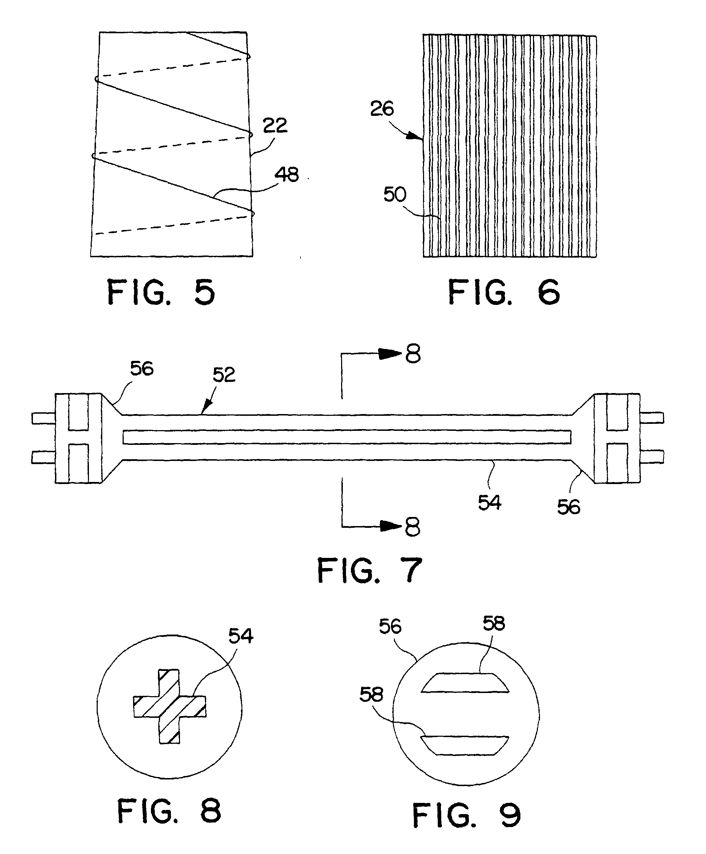

Fig. 5 is a diagrammatic illustration of the fabrication of the second fiber bundle 28 that forms the membrane oxygenator of the preferred embodiment 10. The second fiber bundle 28 comprises thousands of discrete fibers 48 wound in spiral fashion from the top to the bottom and then back again around the heat exchanger housing 22. This is illustrated diagrammatically by the solid and dashed lines in Fig. 5 which extend at angles relative to the vertical central axis of the housing 22. Each fiber 48 is made of a micro-porous polymer material as is well known in the art. The microscopic sized pores in the walls of the hollow fibers 48 permit carbon dioxide from the blood surrounding the outside of the fibers to diffuse into the gas mixture inside the hollow fibers. Similarly, oxygen from the gas mixture inside the hollow fibers can diffuse through the microscopic pores into the blood surrounding the outside of the fibers. Oxygenator fiber bundles of this general type are well known and are commercially available from Medtronic Cardiopulmonary of Anaheim, California, U.S.A. under the trademarks MAXIMA and MAXIMA PLUS. See also U.S. Patent No. 4,975,247 of Badolato, et al. assigned to Medtronic, Inc. entitled MASS TRANSFER DEVICE HAVING A MICROPOROUS, SPIRALLY WOUND HOLLOW FIBER MEMBRANE.

Fig. 6 is a diagrammatic illustration of the first fiber bundle 26 which serves as the core of the heat exchanger portion of the blood oxygenator 10. The fiber bundle 26 has a generally cylindrical configuration and comprises approximately five thousand four-hundred vertically (axially) extending hollow fibers 50. Preferably the fibers are provided as a continuous long web of micro-conduit wrapping material in which the fibers are held together by a thin, flexible, horizontally extending woven interconnect (not illustrated). Such wrapping material is commercially available from Mitsubishi Rayon, Co., Ltd. under the designation HFE430-1 Hollow Fiber. This material uses polyethylene fibers. Similar wrapping material is also commercially available from Hoechst Celanese Corporation under the designation Heat Exchanger Fiber Mat. This material uses polypropylene fibers.

The hollow fibers 50 (Fig. 6) of the heat exchanger fiber bundle 26 have an internal diameter which is so small, *e.g.*, four hundred and twenty-eight microns, that the free flow of blood therethrough may be impaired due to the presence of trapped air bubbles. Accordingly, before using the heat exchanger, it is desirable to pass a wetting agent through the fibers 50. The wetting agent may comprise an ampiphilic molecule having one end which is hydrophilic and a second end which is hydrophobic. An example of such a compound is hydrogenated phosphatidyl choline commercially available from Naderman Corporation under the trademark PHOSPOLIPON. This material has a USP grade and an FDA master file number, approving it for human intravenous use.

The micro-conduit wrapping material of the heat exchanger core is wound about a central, vertically orientated elongate spindle 52 (Fig. 7). The spindle 52 has an intermediate segment 54 having a cross-shaped cross-section, as best seen in Fig. 8. The spindle 52 has enlarged driving ends 56 connected to the opposite ends of the intermediate segment 54. Each of the driving ends 56 has a pair of parallel extending ribs 58 (Fig. 9) which are used to lock the spindle into a winding machine (not illustrated). This machine is utilized to wind the micro-conduit wrapping material about the spindle 52. Preferably the micro-conduit wrapping material is compactly wound about the central spindle 52, but without any substantial tension on the web.

Further details regarding the wetting agent and construction of the heat exchanger fiber bundle 26 are set forth in WO97/25079 entitled BLOOD HEAT EXCHANGE SYSTEM EMPLOYING MICRO-CONDUIT filed on January 11, 1996 and assigned to Medtronic, Inc. of Minneapolis, Minnesota, United States of America.

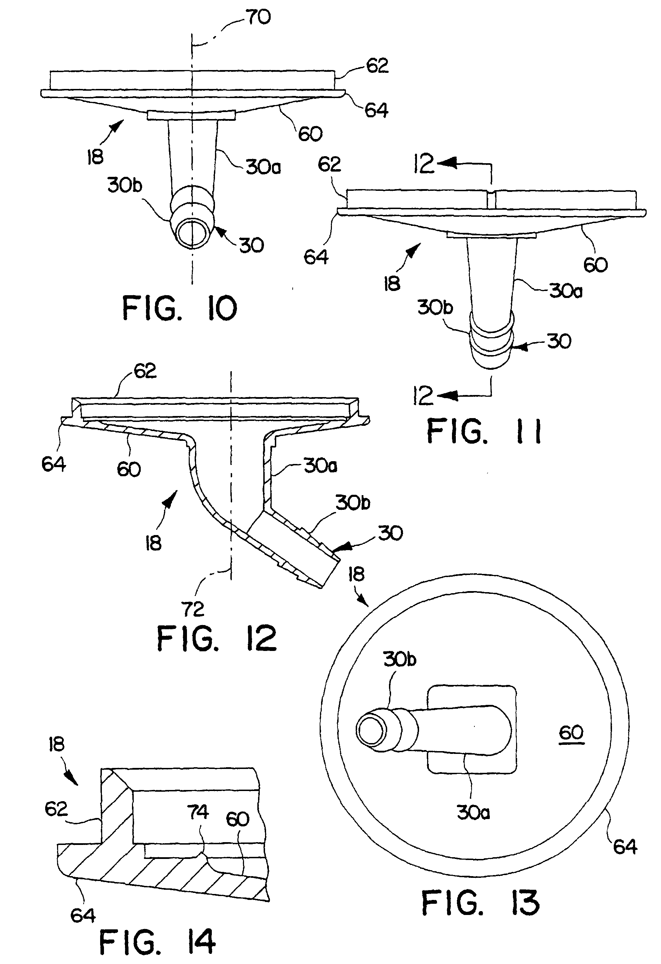

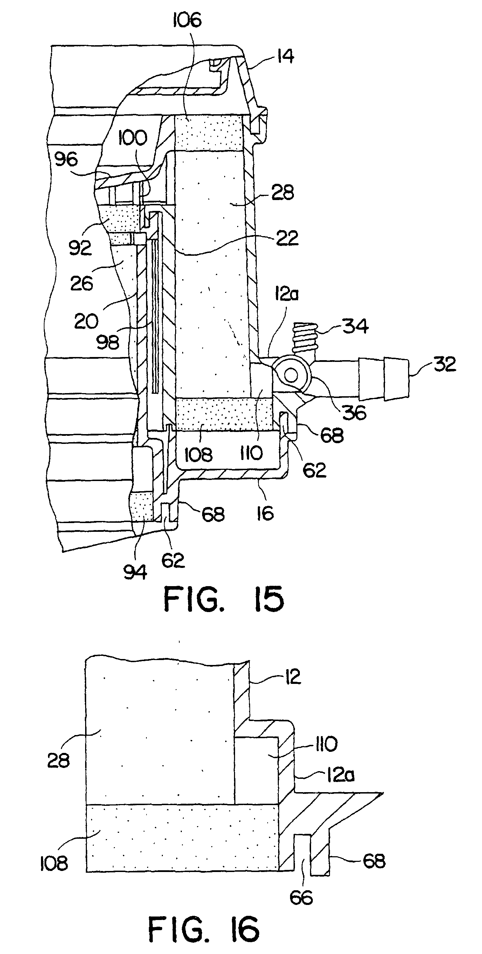

Details of the blood inlet manifold 18 are illustrated in Figs. 10-14. As previously indicated, the blood inlet manifold 18 includes a barbed blood inlet nozzle 30. The nozzle 30 is connected to a piece of flexible elastomeric tubing (not illustrated) which carries oxygen-poor blood from the patient to the blood oxygenator 10. The blood inlet manifold 18 includes a generally conical wall member 60 having a circular vertical lip 62 and a horizontal annular rim 64 surrounding the periphery thereof. The circular vertical lip 62 is configured and dimensioned to be received in a downwardly opening vertical annular recess 66 (Fig. 16). The recess 66 is formed in a downwardly extending annular wall member 68. The wall member 68 is formed with, and projects from, the underside of the lower venous gas header 16. The interfitting relationship of the blood inlet manifold 18 and the raised annular wall member 68 is illustrated in Fig. 15. Preferably the conical wall member 60 (Fig. 10) extends at approximately a ten degree angle relative to a horizontal plane intersecting the vertical axis 70 of the blood oxygenator 10. This axis 70 is illustrated in phantom lines in Fig. 10.

The blood inlet nozzle 30 (Fig. 12) has a downstream segment 30a which extends at approximately a thirty degree angle relative to its upstream barbed segment 30b. The internal configuration of the upstream segment 30b is generally straight and tubular. The upstream segment 30b attaches directly to, and communicates with, the downstream segment 30a. The downstream segment 30a flares outwardly before exiting into the region bordered by the conical wall member 60 and the annular lip 62. A central vertical axis 72 (Fig. 12) of the downstream segment 30a of the inlet nozzle 30 is off center from the central vertical axis 70 (Fig. 10) of the conical wall member 60. A circular raised and pointed projection 74 (Fig. 14) extends upwardly from the outer periphery of the conical wall member 60. It is preferably positioned as close as possible to the co-planar lower ends of the micro-conduits 50 of the heat exchanger fiber bundle 26.

It will be understood that the configuration of the inlet manifold 18 (Figs. 10-14) permits blood to be efficiently distributed from the tubing connected to the barbed nozzle segment 30b into the lower ends of the thousands of individual fibers 50 of the micro-conduit forming the heat exchanger fiber bundle 26. The thirty degree angle between the segments 30a and 30b of the blood inlet nozzle 30 permits the blood oxygenator to be located close to the floor of the surgery room. The tubing carrying the blood from the patient can be connected to the barbed inlet segment 30b and can be gradually bent or curved in the horizontal direction, thereby minimizing the likelihood of kinking.

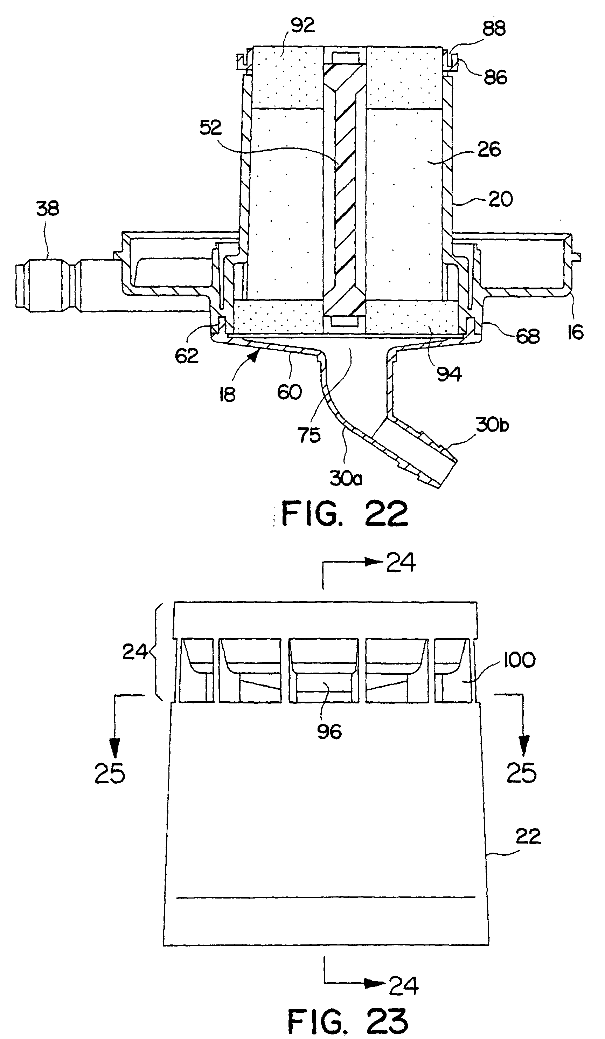

The geometry of the inlet manifold 18 (Figs. 10-14) assures a uniform entry of blood into the thousands of fibers 50 that form the core of the heat exchanger. Nonuniform flow would essentially remove some of the heat exchange surface area from contact with blood. The heat exchanger fiber bundle 26 is compact, measuring, by way of example, approximately 6.25cm (two and one-half inches) in diameter. The internal diameter of the tubing connected to the barbed inlet segment 30b may be, for example, approximately 9.375mm (0.375 inches). Thus, in this example, the blood flood must diverge to almost seven times this diameter in order to uniformly fill the fibers 50 of the heat exchanger fiber bundle 26. The overall height of the blood inlet manifold 18 may be approximately 4.6875cm (one and seven-eighths inches). The height of the circular vertical lip 62 is approximately 7.8125mm (five-sixteenths inches). In this example, the overall vertical height of the chamber 75 (Fig. 22) defined by the conical wall member 60 is about 6.25mm (one-quarter of an inch). The upper and lower ends of the conduits 50 of the heat exchanger fiber bundle 26 terminate in co-planar fashion. The chamber 75 is bounded by the co-planar cut off lower ends of the micro-conduits 50 and the conical wall member 60. The blood inlet nozzle 30 and the conical wall member 60 are configured and dimensioned to permit a blood flow rate of approximately five to seven liters per minute while minimizing shear forces and turbulence that would otherwise traumatize a significant number of cells and platelets in the blood.

The design of the configuration of the blood inlet manifold 30 was facilitated by a computer program based on computational fluid dynamics. The flared configuration of the upstream segment 30a (Fig. 12) of the inlet 30 helps to diverge the blood flow. The ten degree angle of the conical wall member 60 provides for efficient, uniform delivery of blood to the ends of the thousands of fibers of the heat exchanger fiber bundle 26. All this is accomplished with a minimum priming volume and in a manner that minimizes shear forces and recirculation, the presence of which can lead to unacceptable trauma of the blood cells or platelets, and clotting, respectively. The relatively flat angle, *i.e.,* ten degrees, of the conical wall member 60 relative to a horizontal plane extending perpendicular to the vertical axis 70 helps to minimize the priming volume of the blood oxygenator and reduces the number and/or size of recirculations.

Referring to Fig. 12, it can be seen that one side of the upstream segment 30a of the blood inlet 30 is vertical, while the other side follows a complex curve. The misalignment between the center of the inlet segment 30a and the vertical axis 70 has been shown, through computer modeling, to help achieve uniform flow with reduced eddies.

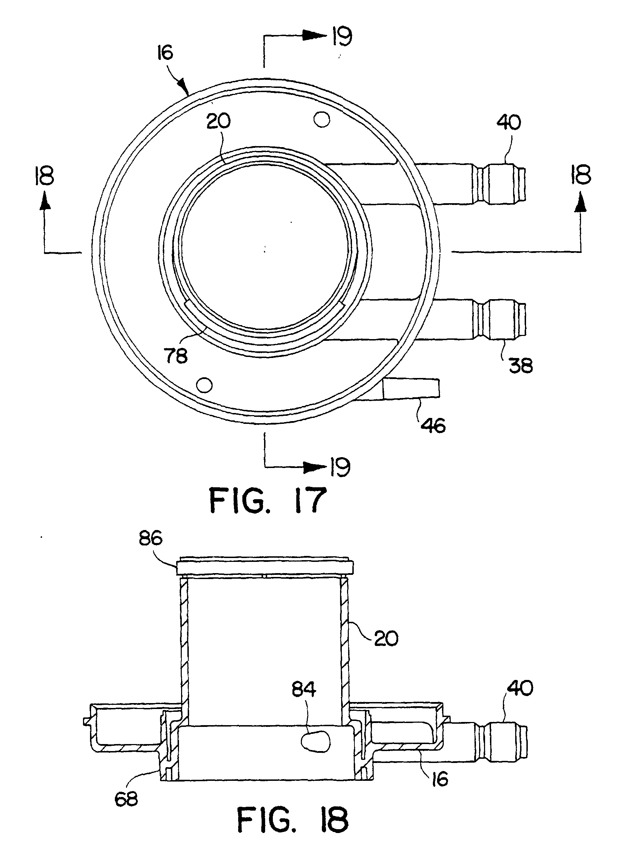

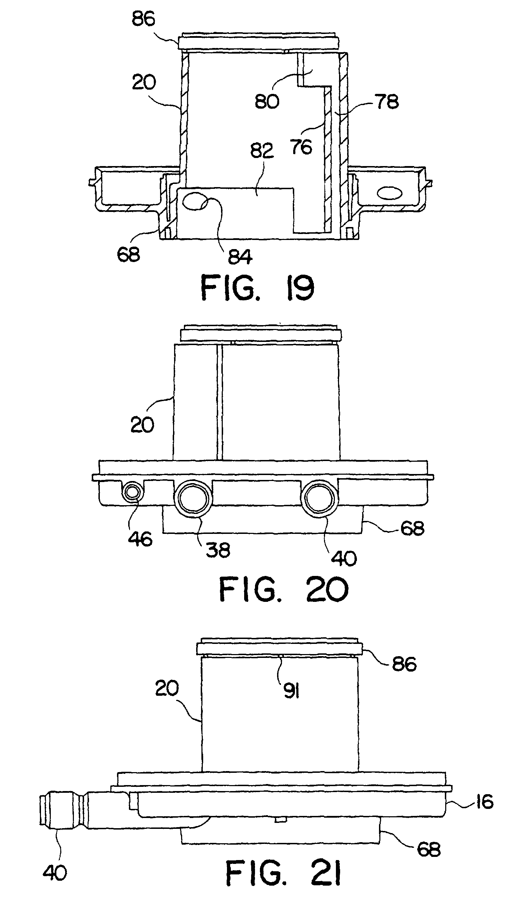

The configuration of the lower venous gas header 16, the inner heat exchanger housing 20, the water inlet and outlet nozzles 38 and 40 and the gas mixture outlet nozzle 46, are illustrated in Figs. 17-21. These parts, along with the raised annular wall member 68 that receives the blood inlet manifold 18, are all injection molded as a single unitary piece of plastic. The inner heat exchanger housing 20 is formed with an interior vertical wall member 76 (Fig. 19) that defines a water flow channel or path 78 (Figs. 17 and 19) which extends vertically along one side of the heat exchanger housing 20. The lower end of the water flow path 78 communicates with the interior of the water inlet nozzle 38. The upper end of the water flow path 78 communicates through a port 80 (Fig. 19) into the upper interior of the housing 20. This permits the incoming heat exchange water to be disbursed around the upper ends of the thousands of micro-conduits or fibers 50 of the heat exchanger fiber bundle 26. As previously explained, this water flows downwardly around the outside of the fibers 50, through another port 82, and then out through water outlet nozzle 40. The opening of the nozzle 40 is shown at 84 in Figure 19.

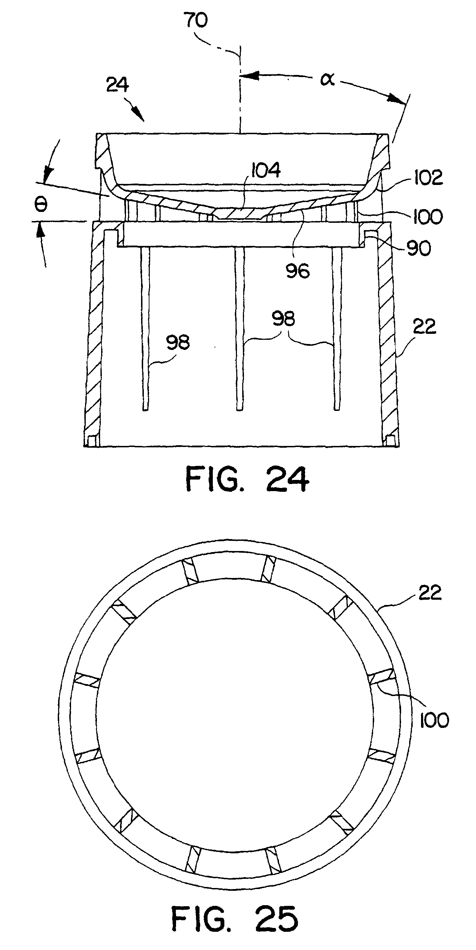

The upper end of the cylindrical heat exchanger housing 20 is molded with a fitting ring 86 (Figs. 19 and 21) having an upwardly opening circular recess 88 (Fig. 22) for receiving, and interfitting with, a downwardly extending circular flange 90 (Fig. 24) of the outer heat exchanger housing 22. The fitting ring 86 is connected to the main part of the housing 20 by small plastic extensions 91 (Fig. 21).

The heat exchanger portion of the blood oxygenator 10 is manufactured in accordance with the following general process. First the micro-conduit wrapping material is wound about the spindle 52 to form the generally cylindrical fiber bundle 26. This fiber bundle is then inserted inside the inner heat exchanger housing 20. Generally disc-shaped bodies 92 and 94 (Fig. 22) of a suitable urethane potting compound are formed at the upper and lower ends of the fiber bundle 26. The potting compound disperses around and between the thousands of fibers at each end. The potting compound also bonds to the inner surface of the housing 20 and to the spindle 52. The bodies 92 and 94 of potting compound therefore form upper and lower water-tight seals. Once the upper and lower seals 92 and 94 have been formed inside the inner heat exchanger housing 20, the ends of the fiber bundle 26 are cleanly cut off in co-planar fashion in order to open the upper and lower ends of the thousands of micro-conduits or fibers 50 in the fiber bundle 26. A suitable wetting agent is preferably applied to the interior surfaces of the micro-conduits or fibers 50 of the fiber bundle 26 as previously indicated. This is done before joining heat exchanger with remaining components of the blood oxygenator.

The reason for providing the seals 92 and 94 is as follows. The water flow passage 78 introduces water into the top of the fiber bundle 26 below the upper seal 92. This water flows downwardly through the fiber bundle 26 around and across the exterior surfaces of the fibers 50 which carry blood upwardly in their minute hollow interiors. The water exits through the outlet nozzle 40 which communicates with the fiber bundle 26 above the lower seal 94. Thus the seals 92 and 94 formed by the urethane potting compound prevent the inter-mixing of blood and water. Where the fibers 50 of the heat exchanger fiber bundle 26 are made of polyethylene or polypropylene, it is desirable to surface treat their ends, e.g. with a corona discharge, in order to enhance the bond between the fibers and the urethane bonding material. Further details of this treatment are described in WO97/25080 entitled SURFACE TREATMENT FOR MICRO-CONDUITS EMPLOYED IN BLOOD HEAT EXCHANGE SYSTEM. That application is also assigned to Medtronic, Inc. of Minneapolis, Minnesota, U.S.A.

Figs. 23-26 illustrate details of the outer heat exchanger housing 22. The housing 22 comprises a generally cylindrical body which incorporates at its upper end a transition manifold 24 including a generally conical wall member 96. The housing 22 has a diameter and height which are selected so that the housing 22 can fit over and around, in concentric fashion, the inner heat exchanger housing 20 as best seen in Figs. 1 and 15. The housing 22 actually is slightly frusto-conical in shape, like vessel 12. Its vertical side wall is slightly tapered, e.g. two degrees. This draft is beneficial when injection molding these components to facilitate ejection from the molding tools. The interior surface of the housing 22 is formed with a plurality of circumferentially spaced, vertically extending tapered ribs 98 (Fig. 24). As previously indicated, the outer heat exchanger housing 22 is formed at its upper end with a circular, downwardly extending flange 90 (Fig. 24) which interfits with, and is received in, an upwardly opening recess 88 in the fitting ring 86 formed at the upper end of the inner heat exchanger housing 20. The transition manifold 24 includes the generally conical wall member 96 (Figs. 23 and 24). It further includes a plurality of radially extending vertical fins 100. The fins 100 are spaced circumferentially about the upper end of the housing 22 and serve to support and connect the conical wall member 96 with the upper end of the main cylindrical shell portion of outer heat exchanger. As illustrated diagrammatically in Fig. 4, the transition manifold 24 serves to redirect the upwardly flowing blood from the micro-conduits or fibers 50 of the heat exchanger fiber bundle 26 radially outwardly around the micro-porous fibers 48 of the oxygenator fiber bundle 28.

The configuration of the transition manifold 24 was also optimized by executing a computer program based on computational fluid dynamics. The configuration of the transition manifold is designed to achieve a uniform distribution of blood flowing out of the heat exchanger fiber bundle 26 into the oxygenator fiber bundle 28, with a minimum of shear forces exerted on the blood cells and platelets. At the same time, the transition manifold 24 enables the blood oxygenator configuration to remain compact, and does not unduly increase the blood volume of the blood oxygenator. Furthermore, the configuration of the transition manifold 24 minimizes shear forces that would otherwise traumatize the blood cells and platelets. It also minimizes re-circulations and stagnant areas that could lead to clotting.

In the preferred embodiment 10 of the blood oxygenator of this invention, the angle 8 (Fig. 24) between the conical wall member 96 and a horizontal plane intersecting the vertical axis 70 is approximately eleven and one half degrees. The transition manifold 24 further includes an upwardly tapered wall section 102 which is circular and is located radially outward from the conical wall member 96. The angle α between the surface of the wall section 102 and the vertical axis 70 is approximately fourteen degrees. The angles θ and α of the wall member 96 and wall section 102 are specifically designed to eliminate recirculations. They also minimize shear forces. The conical wall member 96 includes a central downwardly projecting boss or hub 104 (Fig. 24). This hub 104 has a round configuration and is generally positioned over the center of the heat exchanger fiber bundle 26, adjacent the upper end of its spindle 52. The upper end of the spindle 52 is covered by potting compound. Preferably the hub 104 is positioned as close as possible to the potting compound above the spindle 52 to eliminate a stagnant region that would otherwise exist.

Referring to Figs. 1 and 15, the ring-shaped oxygenator fiber bundle 28 concentrically surrounds the outer heat exchanger housing 22. After the bundle 28 is wound about the housing 22 both components are inserted inside the vessel 12. Upper and lower generally ring-shaped seals 106 and 108 (Fig. 15), respectively, are then formed by introducing a urethane potting compound around the upper and lower ends of the fibers 48 of the oxygenator fiber bundle 28. These seals prevent the blood from flowing into the upper and lower venous gas headers 14 and 16. Thereafter, the upper and lower ends of the fibers 48 are cleanly cut-off to allow the upper and lower hollow interiors of these fibers to communicate with the interior of the upper and lower hollow venous gas headers 14 and 16.

The blood oxygenator 10 of this invention incorporates a specially configured annular blood outlet manifold for collecting the blood from around the fibers 48 at the lower end of the oxygenator fiber bundle 28. More specifically, the flared portion 12a of the vessel 12 (Figs. 1 and 2) provides an annular blood collection chamber 110 (Fig. 16) for collection of the blood and routing of the same through the blood outlet nozzle 32. The chamber 110 has a generally rectangular cross-section, the precise dimensions and configuration of which were determined by executing a computer program based on computational fluid dynamics. The configuration of the blood outlet manifold was designed to uniformly collect blood from the lower portion of the oxygenator fiber bundle 28 and to efficiently route the blood through the blood outlet nozzle 32 with a minimum of shear forces and recirculations. The blood collection chamber 110 (Fig. 15) is formed between the lower outside surface of the oxygenator fiber bundle 28 and the inner wall of the flared portion 12a of the vessel 12.

The vessel 12, and housings 20 and 22 have been described as being generally cylindrical. They actually have a slight degree of taper, e.g. two degrees. In other words, the vertical sidewalls of these structures diverge slightly moving in a downward direction. Thus, it will be understood that the use of the term "generally cylindrical" herein includes minor deviations from perfectly cylindrical.

Except for the fiber bundles 26 and 28, and the potting compound comprising the seals 92, 94, 106 and 108, the remainder of the structures illustrated and described herein are preferably injection molded of clear polycarbonate plastic. Suitable plastics are commercially available under the designations BAYER Makrolon and General Electric LEXAN HP2R-1112. The separately molded plastic components may be assembled and permanently affixed to each other with a suitable non-toxic ultraviolet (UV) curable adhesive.

## Claims

1. A blood oxygenator, comprising:
a heat exchanger including a housing (20,22) and a plurality of conduits (26) having first and second ends, and extending vertically through an interior of the housing;
a first seal (92) surrounding the exterior surfaces of the first ends of the conduits and a second seal (94) surrounding the exterior surfaces of the second ends of the conduits, the first and second seals engaging an inner surface of the heat exchanger housing (20,22) to prevent a heat transfer fluid flowing around the exterior surfaces of the conduits (26) within the interior of the housing from mixing with blood flowing through the conduits;
a heat transfer fluid inlet (38) communicating with the interior of the heat exchanger housing;
a heat transfer fluid outlet (40) communicating with the interior of the heat exchanger housing;
a blood inlet manifold (18) for delivering blood from a first tube (30) to the first ends of the conduits of the heat exchanger;
a generally cylindrical oxygenator fiber bundle (28) concentrically surrounding the heat exchanger; and
a vessel (12) surrounding the oxygenator fiber bundle; **characterised by**
a third seal (106) surrounding the exterior surfaces of the first ends of a plurality of hollow fibers of the oxygenator bundle (28) and a fourth seal (108) surrounding the exterior surfaces of the second ends of the fibers of the oxygenator bundle, the third and fourth seals engaging the housing of the heat exchanger and the vessel to prevent blood flowing around the exterior surfaces of the fibers of the oxygenator bundle from communicating with the hollow interiors of the fibers of the oxygenator fiber bundle;
a transition manifold (24) for delivering blood from the second ends of the conduits of the heat exchanger around the exterior surfaces of the fibers of the oxygenator fiber bundle in a first portion of the oxygenator bundle;
an outlet manifold (12a) for delivering blood from around the exterior surfaces of the fibers of the oxygenator fiber bundle in a second portion of the oxygenator fiber bundle to a second tube;
an inlet header (14) coupled to the vessel for directing a venous gas mixture from a first hose to the first ends of the hollow fibers of the oxygenator fiber bundle so that the gas mixture flows therethrough; and
an outlet header (16) coupled to the vessel for directing the venous gas mixture flowing out of the second ends of the hollow fibers of the oxygenator bundle to a second hose.

2. A blood oxygenator according to Claim 1 in which the conduits of the heat exchanger comprise a web of micro-conduit wrapping material wound into a cylinder.

3. A blood oxygenator according to Claim 1 or 2 in which the housing of the heat exchanger includes an inner generally cylindrical housing (20) and an outer generally cylindrical housing (22) surrounding the inner housing.

4. A blood oxygenator according to Claim 3 in which the transition manifold (24) is formed on an end of the outer housing adjacent to the second ends of the conduits of the heat exchanger.

5. A blood oxygenator according to any preceding Claim in which the first and second seals (92,94) are made of a potting compound.

6. A blood oxygenator according to any preceding Claim in which the third and fourth seals (106,108) are made of a potting compound.

7. A blood oxygenator according to any preceding Claim in which the blood inlet manifold (18) has an inlet nozzle (30) communicating with a region defined by a generally flat conical wall member (60) for uniformly spreading incoming blood from the inlet nozzle over the first ends of the conduits of the heat exchanger.

8. A blood oxygenator according to any preceding Claim in which the transition manifold (24) includes a generally flat conical wall member (68) for directing blood flowing out of the second ends of the conduits radially outwardly to the first ends of the fibers of the oxygenator fiber bundle.

9. A blood oxygenator according to any preceding Claim in which the heat exchanger housing (20, 22) includes means for defining a heat transfer flow path that causes the heat transfer fluid to flow around the outside surfaces of the conduits in a direction substantially opposite to a direction of the flow of blood through the conduits.

10. A blood oxygenator according to any preceding Claim in which the outlet manifold includes a flared portion (12a) of the vessel adjacent the second ends of the hollow fibers of the oxygenator bundle.

11. A method of oxygenating blood while maintaining a predetermined temperature thereof comprising the steps of:
pumping blood in a first direction into the first ends of a plurality of vertical hollow heat exchanger conduits (26) having first and second ends;
pumping a heat transfer fluid around the outside surfaces of the heat exchanger conduits in a second direction opposite to the first direction to transfer heat between the fluid and the blood;
directing the blood out of the second ends of the plurality of hollow conduits in a third direction substantially orthogonal to the first direction generally around a first end portion of the oxygenator fiber bundle (28) and then in the second direction around the outside surfaces of a plurality of hollow fibers of a membrane-type oxygenator fiber bundle concentrically surrounding the heat exchanger conduits;
introducing an oxygen-rich gas mixture into the first ends of the hollow fibers of the oxygenator fiber bundle;
collecting oxygenated blood from around a second end portion of the oxygenator fiber bundle opposite the first end portion; and
collecting a carbon-dioxide enriched gas mixture from the second ends of the hollow fibers of the oxygenator fiber bundle; and further comprising the step of providing seals (92, 94) to separate the blood and the heat transfer fluid; and further comprising the step of providing seals (106, 108) to separate the blood and the gas mixture.

12. A method according to Claim 11 in which the seals are provided by surface treating the outside surfaces of the first and second ends of the conduits with a corona discharge before fabricating the seals by surrounding the outside surfaces with a urethane potting compound.

13. A method according to Claims 12 or 13 and further comprising the step of spreading blood from a tube so that the blood enters substantially all of the first hollow ends of the conduits in a substantially uniform manner.

14. A method according to any of Claim 11 to 13 in which the blood is directed between the second ends of the conduits (26) and the oxygenator fiber bundle (28) so that the blood is dispersed around the first end portion of the oxygenator fiber bundle in a substantially uniform manner.

15. A method according to any of Claims 11 to 14 in which the conduits (26) are made of a polymer material and the inside surfaces thereof are primed with a wetting agent before the blood is pumped therethrough.

16. A method according to any of Claims 11 to 15 and further comprising the step of removing any bubbles in the blood with a de-bubbler coupled to a vessel surrounding the oxygenator fiber bundle.

17. A method according to any of Claims 11 to 16 and further comprising the step of introducing the gas mixture adjacent the first end portion of the oxygenator fiber bundle (28) and venting the gas mixture adjacent the second end portion of the oxygenator fiber bundle.

## Patentansprüche

1. Blutoxygenator mit:
einem Wärmetauscher mit einem Gehäuse (20, 22) und einer Anzahl von Konduits bzw. Kanälen (26), welche erste und zweite Enden aufweisen und sich durch ein Inneres des Gehäuses vertikal erstrecken;
einer ersten Dichtung (92), welche die äußeren Oberflächen der ersten Enden der Konduits umgibt, und einer zweiten Dichtung (94), welche die äußeren Oberflächen der zweiten Enden der Konduits umgibt, wobei die erste und zweite Dichtung eine innere Oberfläche des Wärmetauscher-Gehäuses (20, 22) beaufschlagen bzw. angreifen, um ein Vermischen eines Wärmeübertragungsfluids, welches um die äußeren Oberflächen der Konduits (26) im Inneren des Gehäuses fließt, mit Blut, das durch die Konduits fließt, zu verhindern;
einem Einlass für das Wärmeübertragungsfluid (38), der mit dem Inneren des Wärmetauscher-Gehäuses kommuniziert;
einem Auslass für das Wärmeübertragungsfluid (40), der mit dem Inneren des Wärmetauscher-Gehäuses kommuniziert;
einem Bluteinlassverteiler (18) zum Fördern von Blut von einem ersten Rohr (30) zu den ersten Enden der Konduits des Wärmetauschers;
einem im Allgemeinen zylindrischen Oxygenator-Faserbündel (28), welches den Wärmetauscher konzentrisch umgibt; und
einem Hohlgefäß (12), welches das Oxygenator-Faserbündel umgibt; **gekennzeichnet durch**
eine dritte Dichtung (106), welche die äußeren Oberflächen der ersten Enden einer Anzahl von Hohlfasern des Oxygenator-Bündels (28) umgibt, und eine vierte Dichtung (108), welche die äußeren Oberflächen der zweiten Enden der Fasern des Oxygenator-Bündels umgibt, wobei die dritte und vierte Dichtung das Wärmetauscher-Gehäuse und das Hohlgefäß angreifen, um ein Kommunizieren des Blutes, das um die äußeren Oberflächen der Fasern des Oxygenator-Bündels fließt, mit dem jeweiligen Inneren der Hohlfasern des Oxygenator-Faserbündels zu verhindern;
einen Übergangs- bzw. Übertrittsverteiler (24) zum Fördern von Blut von den zweiten Enden der Konduits des Wärmetauschers um die äußeren Oberflächen der Fasern des Oxygenator-Faserbündels in einem ersten Abschnitt des Oxygenator-Bündels;
einen Auslassverteiler (12a) zum Fördern von um die äußeren Oberflächen der Fasern des Oxygenator-Faserbündels befindlichem Blut in einem zweiten Abschnitt des Oxygenator-Faserbündels zu einem zweiten Rohr;
einen Einlasssammler (14), der für das Leiten eines venösen Gasgemisches aus einem ersten Schlauch zu den ersten Enden der Hohlfasern des Oxygenator-Faserbündels an das Hohlgefäß angeschlossen ist, so dass das Gasgemisch hierdurch fließt; und
einen Aulasssammler (16), der für das Leiten des venösen Gasgemisches, welches aus den zweiten Enden der Hohlfasern des Oxygenator-Bündels fließt, zu einem zweiten Schlauch an das Hohlgefäß angeschlossen ist.

2. Blutoxygenator nach Anspruch 1, bei dem die Konduits des Wärmetauschers ein Netz aus mit Mikro-Konduits ausgestattetem Wickel- bzw. Verpackungsmaterial aufweisen, das zu einem Zylinder aufgewickelt ist.

3. Blutoxygenator nach Anspruch 1 oder 2, bei dem das Wärmetauscher-Gehäuse ein inneres, im Allgemeinen zylindrisches Gehäuse (20) und ein äußeres, im Allgemeinen zylindrisches Gehäuse (22), welches das innere Gehäuse umgibt, aufweist.

4. Blutoxygenator nach Anspruch 3, bei dem der Übergangsverteiler (24) an einem Ende des äußeren Gehäuses angrenzend an die zweiten Enden der Konduits des Wärmetauschers ausgebildet ist.

5. Blutoxygenator nach einem der vorhergehenden Ansprüche,bei dem die erste und zweite Dichtung (92, 94) aus einer Vergussmasse gefertigt sind.

6. Blutoxygenator nach einem der vorhergehenden Ansprüche, bei dem die dritte und vierte Dichtung (106, 108) aus einer Vergussmasse gefertigt sind.

7. Blutoxygenator nach einem der vorhergehenden Ansprüche, bei dem der Bluteinlageverteiler (18) eine Einlassdüse (30) aufweist, die mit einem durch einen im Allgemeinen flachen, konischen Wandabschnitt (60) begrenzten Bereich kommuniziert, so dass eintretendes Blut aus der Einlassdüse gleichmäßig über die ersten Enden der Konduits des Wärmetauschers verteilt wird.

8. Blutoxygenator nach einem der vorhergehenden Ansprüche, bei dem der Übergangsverteiler (24) einen im Allgemeinen flachen konischen Wandabschnitt (68) aufweist, welcher Blut, das aus den zweiten Enden der Konduits heraus fließt, radial nach außen zu den ersten Enden der Fasern des Oxygenator-Faserbündels leitet.

9. Blutoxygenator nach einem der vorhergehenden Ansprüche, bei dem das Wärmetauscher-Gehäuse (20, 22) Mittel aufweist, die einen Wärmeübertragungsfließweg definieren, wodurch ein Fließen des Wärmeübertragungsfluids um die äußeren Oberflächen der Konduits in einer einer Fließrichtung des Blutes durch die Konduits im Wesentlichen entgegengesetzten Richtung bewirkt wird.

10. Blutoxygenator nach einem der vorhergehenden Ansprüche, bei dem der Auslassverteiler einen konisch erweiterten Bereich (12a) des Hohlgefäßes aufweist, der an die zweiten Enden der Hohlfasern des Oxygenator-Faserbündels angrenzt.

11. Verfahren zur Oxygenierung bzw. zur Anreicherung von Blut mit Sauerstoff unter Aufrechterhaltung einer vorgegebenen Temperatur des Blutes mit folgenden Schritten:
Pumpen von Blut in einer ersten Richtung in die ersten Enden einer Anzahl vertikaler, hohler Wärmetauscher-Konduits (26), welche erste und zweite Enden aufweisen;
Pumpen eines Wärmeübertragungsfluids um die äußeren Oberflächen der Wärmetauscher-Konduits in einer zweiten, der ersten Richtung entgegengesetzten Richtung, um Wärme zwischen dem Fluid und dem Blut zu übertragen;
Leiten des Blutes aus den zweiten Enden der Anzahl hohler Konduits in eine dritte, zur ersten Richtung im Wesentlichen orthogonalen Richtung, im Allgemeinen um einen ersten Endabschnitt des Oxygenator-Faserbündels (28), und anschließend in die zweite Richtung, um die äußeren Oberflächen einer Anzahl von Hohlfasern eines Oxygenator-Faserbündels vom Membran-Typ, das die Wärmetauscher-Konduits konzentrisch umgibt;
Einführen eines sauerstoffreichen Gasgemisches in die ersten Enden der Hohlfasern des Oxygenator-Faserbündels;
Sammeln von mit Sauerstoff angereichertem, um einen zweiten Endabschnitt des Oxygenator-Faserbündels befindlichen Blutes, wobei der zweite Endabschnitt dem ersten Endabschnitt gegenüberliegt; und
Sammeln eines mit Kohlendioxid angereicherten Gasgemisches von den zweiten Enden der Hohlfasern des Oxygenator-Faserbündels; ferner mit dem Schritt, Dichtungen (92, 94) bereitzustellen, um das Blut und das Wärmetransferfluid zu trennen; und ferner mit dem Schritt, Dichtungen (106, 108) bereitzustellen, um das Blut und das Gasgemisch zu trennen.

12. Verfahren nach Anspruch 11, bei dem die Dichtungen bereitgestellt werden, indem eine Oberflächenbehandlung der äußeren Oberflächen der ersten und zweiten Enden der Konduits durch eine Koronaentladung durchgeführt wird, und anschließend die Dichtungen durch ein Umgeben der äußeren Oberflächen mit einer Urethan-Vergussmasse hergestellt werden.

13. Verfahren nach einem der Ansprüche 12 oder 13, ferner mit dem Schritt, Blut aus einem Rohr so zu verteilen, dass das Blut im Wesentlichen in alle ersten hohlen Enden der Konduits in einer im Wesentlichen gleichförmigen Art eintritt.

14. Verfahren nach einem der Ansprüche 11 bis 13, bei dem das Blut zwischen den zweiten Enden der Konduits (26) und dem Oxygenator-Faserbündel (28) so geleitet wird, dass das Blut um den ersten Endabschnitt des Oxygenator-Faserbündels in einer im Wesentlichen gleichförmigen Art verteilt wird.

15. Verfahren nach einem der Ansprüche 11 bis 14, bei dem die Konduits (26) aus einem Polymer gefertigt sind, und deren innere Oberflächen mit einem Benetzungsmittel vorbehandelt werden, bevor das Blut hindurch gepumpt wird.

16. Verfahren nach einem der Ansprüche 11 bis 15, ferner mit dem Schritt, jegliche Bläschen im Blut mit einem Blasenentferner zu entfernen, der an ein Hohlgefäß angeschlossen ist, welches das Oxygenator-Faserbündel umgibt.

17. Verfahren nach einem der Ansprüche 11 bis 16, ferner mit dem Schritt, das Gasgemisch angrenzend an den ersten Endabschnitt des Oxygenator-Faserbündels (28) einzuführen, und das Gasgemisch angrenzend an den zweiten Endabschnitt des Oxygenator-Faserbündels abzulassen.

## Revendications

1. Oxygénateur de sang, comprenant :
un échangeur thermique comprenant un boîtier (20, 22) et une pluralité de conduits (26) ayant des première et deuxième extrémités, et s'étendant verticalement à travers l'intérieur du boîtier ;
un premier joint d'étanchéité (92) entourant les surfaces extérieures des premières extrémités des conduits et un deuxième joint d'étanchéité (94) entourant les surfaces extérieures des deuxièmes extrémités des conduits, les premier et deuxième joints d'étanchéité mettant en prise une surface interne du boîtier d'échangeur thermique (20, 22) pour empêcher un fluide de transfert thermique circulant autour des surfaces extérieures des conduits (26) à l'intérieur du boîtier de se mélanger avec le sang circulant dans les conduits ;
une entrée de fluide de transfert thermique (38) communiquant avec l'intérieur du boîtier échangeur thermique ;
une sortie de fluide de transfert thermique (40) communiquant avec l'intérieur du boîtier d'échangeur thermique ;
un collecteur d'entrée de sang (18) pour délivrer du sang depuis un premier tube (30) aux premières extrémités des conduits de l'échangeur thermique ;
un faisceau de fibres d'oxygénation généralement cylindrique (28) entourant de manière concentrique l'échangeur thermique ; et
une cuve (12) entourant le faisceau de fibres d'oxygénation ; **caractérisé par**
un troisième joint d'étanchéité (106) entourant les surfaces extérieures des premières extrémités d'une pluralité de fibres creuses du faisceau d'oxygénation (28) et un quatrième joint d'étanchéité (108) entourant les surfaces extérieures des deuxièmes extrémités des fibres du faisceau d'oxygénation, les troisième et quatrième joints d'étanchéité mettant en prise le boîtier de l'échangeur thermique et la cuve pour empêcher le sang circulant autour des surfaces extérieures des fibres du faisceau d'oxygénation de communiquer avec les intérieurs creux des fibres du faisceau de fibres d'oxygénation ;
un collecteur de transition (24) pour distribuer du sang depuis les deuxièmes extrémités des conduits de l'échangeur thermique autour des surfaces extérieures des fibres du faisceau de fibres d'oxygénation dans une première partie du faisceau d'oxygénation ;
un collecteur de sortie (12a) pour distribuer du sang depuis autour des surfaces extérieures des fibres du faisceau de fibres d'oxygénation dans une deuxième partie du faisceau de fibres d'oxygénation vers un deuxième tube ;
une tête d'entrée (14) couplée à la cuve pour orienter un mélange de gaz veineux depuis un premier tuyau flexible vers les premières extrémités des fibres creuses du faisceau de fibres d'oxygénation de sorte que le mélange de gaz circule à travers celui-ci ; et
une tête de sortie (16) couplée à la cuve pour orienter le mélange de gaz veineux s'écoulant par les deuxièmes extrémités des fibres creuses du faisceau d'oxygénation vers un deuxième tuyau flexible.

2. Oxygénateur de sang selon la revendication 1 dans lequel les conduits de l'échangeur thermique comprennent une bande de matériau d'emballage à micro-conduits enroulée dans un cylindre.

3. Oxygénateur de sang selon la revendication 1 ou 2 dans lequel le boîtier de l'échangeur thermique comprend un boîtier interne généralement cylindrique (20) et un boîtier externe généralement cylindrique (22) entourant le boîtier interne.

4. Oxygénateur de sang selon la revendication 3 dans lequel le collecteur de transition (24) est formé sur une extrémité du boîtier externe contiguë aux deuxièmes extrémités des conduits de l'échangeur thermique.

5. Oxygénateur de sang selon l'une quelconque des revendications précédentes dans lequel les premier et deuxième joints d'étanchéité (92, 94) sont constitués d'un composé de remplissage.

6. Oxygénateur de sang selon l'une quelconque des revendications précédentes dans lequel les troisième et quatrième joints d'étanchéité (106, 108) sont constitués d'un composé de remplissage.

7. Oxygénateur de sang selon l'une quelconque des revendications précédentes dans lequel le collecteur d'entrée de sang (18) a une buse d'entrée (30) communiquant avec une région définie par un élément de paroi conique généralement plat (60) pour répandre uniformément le sang provenant de la buse d'entrée sur les premières extrémités des conduits de l'échangeur thermique.

8. Oxygénateur de sang selon l'une quelconque des revendications précédentes dans lequel le collecteur de transition (24) comprend un élément de paroi conique généralement plat (68) pour orienter le sang s'écoulant hors des deuxièmes extrémités des conduits radialement vers l'extérieur vers les premières extrémités des fibres du faisceau de fibres d'oxygénation.

9. Oxygénateur de sang selon l'une quelconque des revendications précédentes dans lequel le boîtier d'échangeur thermique (20, 22) comprend des moyens pour définir un trajet d'écoulement de transfert thermique qui conduit le fluide de transfert thermique à circuler autour des surfaces extérieures des conduits dans une direction sensiblement opposée à la direction de l'écoulement de sang dans les conduits.

10. Oxygénateur de sang selon l'une quelconque des revendications précédentes dans lequel le collecteur de sortie comprend une partie élargie (12a) de la cuve contiguë aux deuxièmes extrémités des fibres creuses du faisceau d'oxygénation.

11. Procédé d'oxygénation du sang tout en maintenant une température prédéterminée de celui-ci comprenant les étapes consistant à :
pomper du sang dans une direction dans les premières extrémités d'une pluralité de conduits d'échangeur thermique creux verticaux (26) ayant des premières et deuxièmes extrémités ;
pomper un fluide de transfert thermique autour des surfaces extérieures des conduits d'échangeur thermique dans une deuxième direction opposée à la première direction pour transférer la chaleur entre le fluide et le sang ;
conduire le sang hors des deuxièmes extrémités de la pluralité de conduits creux dans une troisième direction sensiblement orthogonale à la première direction généralement autour d'une partie de première extrémité du faisceau de fibres d'oxygénation (28), puis dans la deuxième direction autour des surfaces extérieures d'une pluralité de fibres creuses d'un faisceau de fibres d'oxygénation de type à membrane entourant de manière concentrique les conduits d'échangeur thermique ;
introduire un mélange de gaz riche en oxygène dans les premières extrémités des fibres creuses du faisceau de fibres d'oxygénation ;
collecter le sang oxygéné depuis autour d'une partie de deuxième extrémité du faisceau de fibres d'oxygénation opposée à la partie de première extrémité ; et
collecter un mélange de gaz enrichi en dioxyde de carbone depuis les deuxièmes extrémités des fibres creuses du faisceau de fibres d'oxygénation ; et comprenant, de plus, l'étape consistant à fournir des joints d'étanchéité (92, 94) pour séparer le sang et le fluide de transfert thermique ; et
comprenant, de plus, l'étape consistant à fournir des joints d'étanchéité (106, 108) pour séparer le sang et le mélange de gaz.

12. Procédé selon la revendication 11 dans lequel les joints d'étanchéité sont formés par traitement superficiel des surfaces extérieures des première et deuxième extrémités des conduits avec une décharge corona avant de fabriquer les joints d'étanchéité en entourant les surfaces extérieures avec un composé de remplissage d'uréthane.

13. Procédé selon les revendications 12 ou 13 et comprenant, de plus, l'étape consistant à répandre le sang provenant d'un tube de telle manière que le sang entre dans à peu près toutes les premières extrémités creuses des conduits d'une manière sensiblement uniforme.

14. Procédé selon l'une quelconque des revendications 11 à 13 dans lequel le sang est conduit entre les deuxièmes extrémités des conduits (26) et le faisceau de fibres d'oxygénation (28) de telle manière que le sang soit dispersé autour de la partie de première extrémité du faisceau de fibres d'oxygénation d'une manière sensiblement uniforme.

15. Procédé selon l'une quelconque des revendications 11 à 14 dans lequel les conduits (26) sont constitués d'un matériau polymère et les surfaces intérieures de ceux-ci sont amorcées avec un agent mouillant avant que du sang soit pompé dans ceux-ci.

16. Procédé selon l'une quelconque des revendications 11 à 15 et comprenant, de plus, l'étape consistant à éliminer les bulles dans le sang avec un débulleur couplé à une cuve entourant le faisceau de fibres d'oxygénation.

17. Procédé selon l'une quelconque des revendications 11 à 16 comprenant, de plus, l'étape consistant à introduire le mélange de gaz en position contiguë à la partie de première extrémité du faisceau de fibres d'oxygénation (28) et la purge du mélange de gaz en position contiguë à la partie de deuxième extrémité du faisceau de fibres d'oxygénation.
